# EUROPEAN PATENT SPECIFICATION

(11) **EP 1 743 610 B1**
(45) Date of publication and mention of the grant of the patent: **25.03.2009**
(21) Application number: 05015069.7
(22) Date of filing: 12.07.2005
(51) Int. Cl.: A61F 13/49

(54) **Low cost diaper with reduced sagging**
Preisgünstige Windel mit reduzierter Neigung durchzuhängen
Couche-culotte bonmarché à affaissement reduit

(43) Date of publication of application: 17.01.2007
(73) Proprietor: The Procter and Gamble Company, Cincinnati, Ohio 45202 (US)
(72) Inventor: Endres, Joerg, 35428 Langgoens (DE); Stoelzel, Claus-Peter, 65812 Bad Soden (DE)
(74) Representative: Borbach, Markus

(56) References cited:
- US-A- 4 883 480
- US-A- 5 782 819

## Description

### FIELD OF THE INVENTION

A common problem associated with disposable diapers is to achieve optimized fit on the wearer. Especially, the diaper has to provide good fit around the waist and hip of the wearer in order to prevent sagging and drooping during use. At the same time, the pressure exerted onto the waist and hip should not become excessively high, such that the diaper becomes uncomfortable for the wearer.

This problem has been addressed for example in U.S. 5,358,500 entitled "Absorbent articles providing sustained dynamic fit" which issued to Lavon et al. on October 25, 1994. In U.S. 5,358,500 a disposable diaper is disclosed, which comprises a closure system designed so that a primary line of tension fitting at an angle to the body, is formed to secure the absorbent article on a wearer in a manner that does not contribute to sagging and sliding of the diaper. Preferably, this is achieved by providing angled tapes and an angled landing zone.

U.S. Patent 5,899,896 entitled "Absorbent article with fastening system to prevent drooping" which issued to Suprise et al. on May 4, 1999 also refers to absorbent articles which are configured to prevent drooping and to improve fit. U.S. 5,899,896 discloses an absorbent article which comprises attachment means for attaching the article about the waist of a wearer such that attachment points are located on the sides of the article and behind a transverse center plane of the article.

Another way to improve the fit of the diaper around the waist and hip of the wearer is to imply at least partial elasticity to the diaper. One common way of implying partial elasticity is the provision of elastically stretchable back ear panels. Elastically stretchable ear panels are disclosed e.g. in U.S. 6,575,949 entitled "Perforated stretch ear diaper" which issued to Waksmundzki et a. on June 10, 2003. However, the use of elastically stretchable materials considerably adds to the overall costs of the article due to increased material- and manufacturing costs.

Hence, there is still a need for a diaper with good fit, especially with respect to the prevention of sagging and drooping of the article, wherein the article can be produced in a cost-effective manner. It is an objective of the present invention to provide such diapers.

### SUMMARY OF THE INVENTION

The present invention relates to a disposable diaper having a front waist region, a back waist region and a crotch region between the front waist region and the back waist region. The diaper comprises a chassis having longitudinal edges and end edges and comprising a liquid pervious topsheet, a liquid impervious backsheet associated with the topsheet, and an absorbent core disposed between the topsheet and the backsheet.

The diaper further comprises first and second back ear panels extending laterally outward from the chassis in the back waist region. Each of the back ear panels have an innermost longitudinal edge line and an outermost longitudinal edge line; wherein each of the back ear panels is joined along its innermost longitudinal edge line to a respective longitudinal edge of the chassis with each of the back ear panels being non-elastic.

The chassis has a longitudinal length L1 and the first and second back ear panel each have a longitudinal length L2. The ratio of L2 to L1 is from 0.18 to 0.24.

The diaper further comprises a fastening system comprising a pair of tapes each having a longitudinal width W1, wherein one tape is attached to the first back ear panel and the other tape is attached to the second back ear panel, the tapes extending laterally outwardly from the back ear panels beyond the outermost longitudinal edge line of the back ear panels. Each tape has a width W1 from 28 mm to 40 mm.

### BRIEF DESCRIPTION OF THE DRAWINGS

While the specification concludes with claims pointing out and distinctly claiming the present invention, it is believed the same will be better understood by the following drawings taken in conjunction with the accompanying specification wherein like components are given the same reference number.
Figure 1 is a top plan view of a disposable diaper according to an embodiment of the present invention
Figure 2 is a top plan view of a disposable diaper according to another embodiment of the present invention

### DETAILED DESCRIPTION OF THE INVENTION

### Definitions:

As used herein, the following terms have the following meanings:
"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).
"Diaper" refers to an absorbent article generally worn by infants and incontinent persons about the lower torso.
"Comprise," "comprising," and "comprises" is an open ended term that specifies the presence of what follows e.g. a component but does not preclude the presents of other features, elements, steps or components known in the art, or disclosed herein.
An "element" or "material" according to the present invention can consist only of a single layer or can comprise multiple layers. If the element or material comprises multiple layers, the layers may be similar or non-similar.

"Extensible" as used herein refers to an element or material which, upon application of a force, elongates beyond its original length by at least 10 % if subjected to the following test:

A rectangular piece of material or a rectangular piece of the element having a width of 2.54cm (1 inch) and a length of 25.4 cm(10 inch) is maintained in a vertical position by holding the piece along its upper 1 inch wide edge along its complete width.

A force of 10 N is applied onto the opposite lower edge along the complete width of the material for minute at 25°C.

Immediately after one minute, the length of the piece is measured while the force is still applied and the degree of elongation is calculated by subtracting the initial length (25.4cm; 10 inch) from the length measured after one minute.

"Non-extensible" as used herein refers to an element or material which, upon application of a force as described above with respect to "extensible" elongates by no more than 10% beyond its original length. For the present invention, a non-extensible material or element is also considered to be non-elastic.

"Non-elastic" as used herein refers to an element or material which is non-extensible. Further, "non-elastic" also refers to an extensible element or material if the element or material does not recover by more than 20% if subjected to the following test, which is to be carried out immediately subsequent to the test on "extensibility" set out above:

Immediately after the length of the rectangular piece of material/element has been measured while the 10N force is still applied, the force is removed and the piece is laid down flat on a table for 5 minutes (at 25°C) to be able to recover. Immediately after 5 minutes, the length of the piece is measured again and the degree of elongation is calculated by subtracting the initial length (25.4cm; 10 inch) from the length after 5 minutes.

The elongation after one minute while the force has been applied (as measured with respect to "extensibility") is compared to the elongation after the piece has been laid down flat on a table for 5 minutes: If the elongation does not recover by more than 20%, the material or element is considered to be "non-elastic".

"Highly non-elastic" as used herein refers to a material or element, which is either "non-extensible" or which does not recover by more than 10% if subjected to the test set out above for "non-elastic".

"Elastically extensible" refers to an element or material which is extensible and which recovers by at least 20% after elongation if subjected to the test set out above for "non-elastic".

The terms "slippage" and "sagging" are used interchangeably herein and refer to the slippage of the diaper downward from the waist and hip of the wearer.

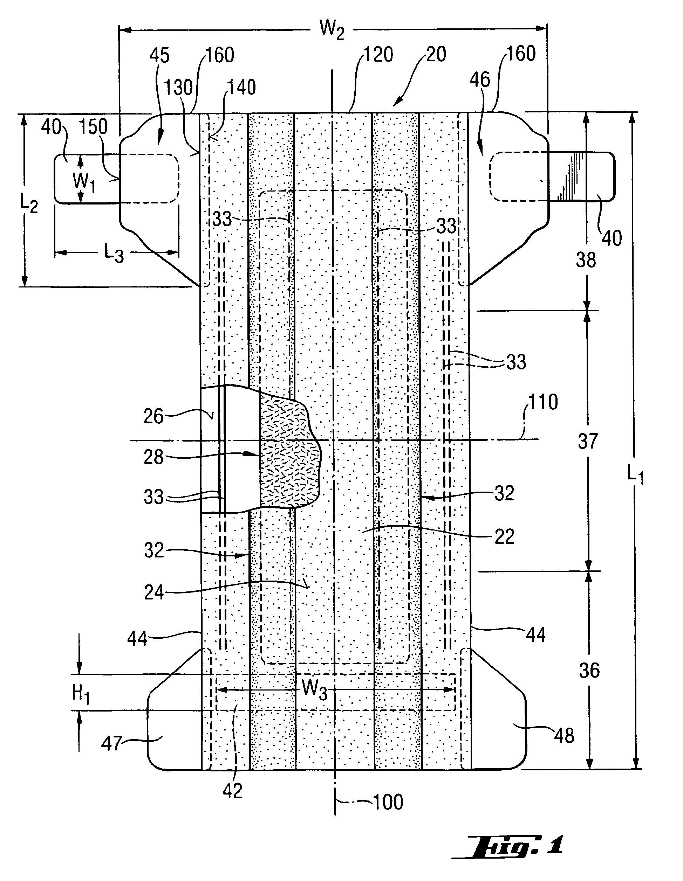

The present invention provides a disposable diaper such as a diaper 20 as shown in Figure 1. Figure 1 is a plan view of a diaper 20 in its flat out, un-contracted state (i.e., without elastic induced contraction). Portions of the structure are cut away to more clearly show the underlying structure of the diaper 20. The diaper 20 has a longitudinal axis 100 and a transverse axis 110. The diaper 20 has further an inner surface (facing the viewer in FIG. 1) and an outer surface opposed to the inner surface. The inner surface of the diaper 20 comprises that portion of the diaper 20 which is positioned adjacent to the wearer's body during use. The outer surface comprises that portion of the diaper 20 which is positioned away from the wearer's body.

One end portion of the diaper 20 is configured as a front waist region 36 of the diaper 20. The opposite end portion is configured as a back waist region 38 of the diaper 20. An intermediate portion of the diaper 20 is configured as a crotch region 37, which extends longitudinally between the front and back waist regions 36 and 38. The crotch region 37 is that portion of the diaper 20 which, when the diaper 20 is worn, is generally positioned between the wearer's legs.

The chassis 22 of the diaper 20 comprises the main body of the diaper 20. The chassis 22 comprises a liquid pervious topsheet 24 and a backsheet 26. The chassis 22 further includes an absorbent core 28 encased between the topsheet 24 and the backsheet 26. The chassis 22 has a periphery which is defined by the outer edges of the chassis 22 with longitudinal edges 130 and end edges 120. The chassis 22 is preferably non-elastic, i.e. the topsheet 24, backsheet 26 and absorbent core 28 are preferably non-elastic. More preferably, the chassis 22 is highly non-elastic, i.e. the topsheet 24, backsheet 26 and absorbent core 28 are preferably highly non-elastic. Moreover, the chassis is preferably non-extensible, i.e. it does not undergo substantial plastic deformation upon elongation. The chassis 22 is preferably rectangular shaped but ―in a less preferred embodiment- may also be slightly hourglass-shaped.

The length L1 of the chassis varies depending on the size of the diaper: Typically, the length L1 of the diaper varies from about 380 mm for the smallest size diapers (size 1) to about 580 mm for the baby diaper with the largest size. Typically, not all the components comprised by the chassis 22 (e.g. the topsheet 24, the backsheet 26 and the absorbent core 28) extend over the whole surface of the chassis 22, especially the absorbent core 28 is commonly smaller in extension than the topsheet 24 and/or the backsheet 26. For the present invention, the length L1 of the chassis 22 is determined as the length of the largest component of all chassis components (i.e. the component with the largest extension in the longitudinal direction of the diaper). If this component is not rectangular in shape, the largest longitudinal extension of this component is taken as length L1. The length L1 is measured along or parallel to the longitudinal centerline 100 from one end edge 120 to the other end edge 120 of the diaper while the diaper is in its flat out, un-contracted state (i.e., without elastic induced contraction after the leg elastics have been cut through).

The width of the preferably rectangular shaped chassis 22 (i.e. the transverse dimension without ear panels attached to the chassis) is preferably from 190 mm to 250 mm.

The topsheet 24 is preferably compliant, soft feeling, and non-irritating to the wearer's skin. Further, the topsheet 24 is liquid pervious permitting liquids (e.g., urine) to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be made of natural fibers (e.g., wood or cotton fibers), synthetic fibers (e.g., polymeric fibers such as polyester, polypropylene, or polyethylene fibers) or from a combination of natural and synthetic fibers. The topsheet 24 is preferably made of a hydrophobic material to isolate the wearer's skin from liquids which have passed through the topsheet 24 and are contained in the absorbent core 28 (i.e., to prevent rewet). If the topsheet 24 is made of a hydrophobic material, preferably at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 28. The topsheet 24 can be rendered hydrophilic by treating it with a surfactant. In a preferred embodiment, the topsheet 24 is a nonwoven web that can provide reduced tendency for surface wetness and consequently facilitate maintaining urine absorbed by the core 28 away from the user's skin, after wetting.

The absorbent core 28 generally is disposed between the topsheet 24 and the backsheet 26. The absorbent core 28 may comprise any absorbent material that is generally compressible, conformable, non-irritating to the wearer's skin, and capable of absorbing and retaining liquids such as urine and other certain body exudates.

The absorbent core 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding, meltblown polymers including coform, cross-linked cellulosic fibers, tissue including tissue wraps and tissue laminates, absorbent foams, absorbent sponges, superabsorbent polymers, absorbent gelling materials, or any equivalent material or combinations of materials. The configuration and construction of the absorbent core may also be varied, e.g. the absorbent core may have varying caliper zones, a hydrophilic gradient, a superabsorbent gradient, or lower average density and lower average basis weight acquisition zones; or may comprise one or more layers or structures. The total absorbent capacity of the absorbent core 28 should, however, be compatible with the design loading and the intended use of the diaper 20. Further, the size and absorbent capacity of the absorbent core 28 may be varied to accommodate wearers ranging from infants through adults.

The backsheet 26 in Figure 1 may be joined with the topsheet 24. The backsheet 26 prevents the exudates absorbed by the absorbent core 28 and contained within the article 20 from soiling other external articles that may contact the diaper 20, such as bed sheets and undergarments. In preferred embodiments, the backsheet 26 is substantially impervious to liquids (e.g., urine) and comprises a thin plastic film such as a thermoplastic film having a thickness of about 0.012 mm (0.5 mil) to about 0.051 mm (2.0 mils). Suitable backsheet films include those manufactured by Tredegar Industries Inc. of Terre Haute, IN and sold under the trade names X15306, X10962, and X10964. Other suitable backsheet materials may include breathable materials that permit vapors to escape from the diaper 20 while still preventing exudates from passing through the backsheet 26. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, TX, under the designation EXXAIRE. Suitable breathable composite materials comprising polymer blends are available from Clopay Corporation, Cincinnati, OH under the name HYTREL blend P 18-3097. Such breathable composite materials are described in greater detail in PCT Application No. WO 95/16746, published on June 22, 1995 in the name of E. I. DuPont and co-pending U.S. Patent Application Serial No. 08/744,487, filed on November 6, 1996 in the name of Curro. Other breathable backsheets including nonwoven webs and apertured formed films are described in U.S. Patent No. 5,571,096 issued to Dobrin et al. on November 5, 1996.

The backsheet 26 may further comprise an outer cover nonwoven layer which is joined with the garment-facing surface of the preferably liquid impervious film to form a laminate. The outer cover nonwoven layer may be formed by any type of nonwoven material. Preferred nonwoven materials include a carded nonwoven material, spunbonded nonwoven material and a meltblown nonwoven material.

The diaper 20 further includes elasticized leg cuffs 32 for providing improved containment of liquids and other body exudates. The elasticized leg cuffs 32 may include several different embodiments for reducing the leakage of body exudates in the leg regions. (The leg cuffs can be and are sometimes also referred to as leg bands, side flaps, barrier cuffs, elastic cuffs or gasketing cuffs.) Preferred elasticized leg cuff designs are disclosed in, for example, U.S. Patent 3,860,003 issued to Buell on January 14, 1975; U.S. Patent 4,695,278 issued to Lawson on September 22, 1987; and U.S. Patent 4,795,454 issued to Dragoo on January 3, 1989. While each elasticized leg cuff 32 may be configured so as to be similar to any of the leg bands, side flaps, barrier cuffs, or elastic cuffs described above, it is preferred that the elasticized leg cuff 33 includes an elastic gasketing cuff with one or more elastic strands 33 which is described in the above-referred U.S. Patent Nos. 4,695,278 and 4,795,454.

The diaper 20 of the present invention further comprises back ear panels 45, 46, which are joined to the chassis 22 along the longitudinal edges 130 of the chassis 22 in the back waist region 38 of the diaper. Thereby, the back ear panels 45, 46 extend laterally outwardly from the chassis 22 in the back waist region 38.

The back ear panels 45, 46 are preferably made of nonwoven material, more preferably of spunbonded nonwoven material. It is preferred that the back ear panels have a basis weight of from 30 gsm to 70 gsm, more preferably from 40 to 50 gsm. The back ear panels are preferably made of polypropylene (PP), of polyethylene (PE) or mixtures thereof. The back ear panels 45, 46 of the present invention are non-elastic, more preferably highly non-elastic and still more preferably they are also non-extensible.

The back ear panels 45, 46 have an innermost longitudinal edge line 140 and an outermost longitudinal edge line 150. Further, the back ear panels 45, 46 have end edges 160. The back ear panels 45, 46 are joined to the chassis 22 along their innermost longitudinal edge line 140. The back ear panels 45, 46 can be joined to the chassis 22 by any suitable method known in the art, such as adhesive bonding, ultrasonic bonding, pressure bonding, heat bonding or any combinations thereof. The back ear panels 45, 46 may be joined in a continuous or in an intermittent manner.

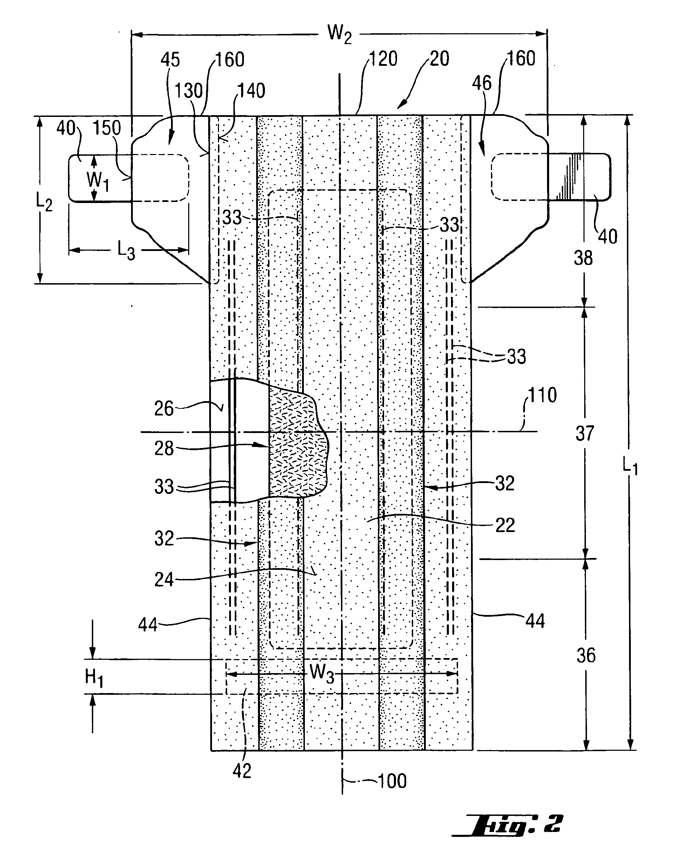

The back ear panels 45, 46 may be joined to the chassis 22 such that the end edge 160 of the back ear panel forms one straight line with the end edge 120 of the chassis in the back waist region (as shown in Figures 1 and 2). Alternatively- e.g. due to ease of the manufacturing process - the back ear panels may be joined slightly below the end edge of the chassis 22, e.g. by a small distance of up to 2 cm, more preferably not more than 1.5 cm.

The diapers of the present invention preferably also comprise a pair of front ear panels 47, 48 joined to the chassis 22 along the longitudinal edge lines 130 of the chassis in the front waist region 36 of the diaper 20. Thereby, the front ear panels 47, 48 extend laterally outward from the chassis 22 in the front waist region 36.

The front ear panels 47, 48 may be joined to the chassis 22 such that the end edge of the front ear panel forms one straight line with the end edge of the chassis in the front waist region (as shown in Figure 1). Alternatively - e.g. due to ease of the manufacturing process - the front ear panels may be joined slightly below the end edge of the chassis 22, e.g. by a small distance of up to 2 cm, more preferably not more than 1.5 cm.

The front ear panels 47, 48 preferably are made of nonwoven material, more preferably of spunbonded nonwoven material. It is preferred that the front ear panels 47, 48 have a basis weight of from 20 to 60 gsm, preferable from 40 to 50 gsm. The front ear panels are preferably made of polypropylene (PP), of polyethylene (PE) or mixtures thereof. The front ear panels 47, 48 of the present invention are non-elastic, more preferably highly non-elastic and still more preferably they are also non-extensible

The front ear panels 47, 48 have an innermost longitudinal edge line and an outermost longitudinal edge line. The front ear panels 47, 48 are joined to the chassis 22 along their innermost longitudinal edge line. The front ear panels 47, 48 can be joined to the chassis 22 by any suitable method known in the art, such as adhesive bonding, ultrasonic bonding, pressure bonding, heat bonding or any combinations thereof. The front ear panels 47, 48 may be joined in a continuous or in an intermittent manner.

In order to keep the diaper 20 in place about the wearer, the diaper 20 further comprises a fastening system. The fastening system comprises a pair of tapes 40. The tapes are attached adjacent the outermost longitudinal edge line 150 of each back ear panel 45, 46 and extend laterally outward from the back ear panels 45, 46 beyond the outermost longitudinal edge line 150.

The fastening system further comprises one landing member 42 disposed on the outer surface of the backsheet 26 in the front waist region 36 of the diaper.

The tapes 40 comprise a first fastening component and the landing member 42 comprises a complimentary second fastening component engageable with the first fastening component 37. An exemplary fastening system wherein the first and second fastening components each comprise mechanical closure elements are hook and loop fastening materials as disclosed e.g. in U.S. Pat. No. 4,963,140 entitled "Mechanical Fastening Systems with Disposal Means for Disposable Absorbent Articles" issued to Robertson and Scripps on Oct. 16, 1990. A fastening system having combination adhesive/mechanical closure elements is described in U.S. Pat. No. 4,946,527 entitled "Pressure-Sensitive Adhesive Fastener and Method of Making Same" issued to Battrell on Aug. 7, 1990. In a preferred embodiment of the present invention the fastening system comprises a mechanical fastening system of the hook and loop type. The first fastening components, comprised by the tapes 40, preferably comprise the hook members. The landing member 42 comprises the corresponding loop material as the second fastening component. The loop material can be made of a laminate of film backing and knitted fabric or, alternatively, can be made of nonwoven material.

The tapes 40 have a proximal portion and a distal portion. The tapes 40 are preferably joined to the outer, garment facing surface of the back ear panels 45, 46 by their proximal portion to create a fixed portion (i.e., that end of the tape 40 permanently joined to the back ear panel 45, 46 of the diaper 20 during manufacture).

The tapes 40 can be joined to the back ear panels by any suitable method known in the art, such as adhesive bonding, ultrasonic bonding, pressure bonding, heat bonding or any combinations thereof.

The distal, free portion of the tape 40 is that portion that extends laterally outwardly beyond the outermost longitudinal edge line 150 of the back ear panel 45, 46.

The tapes 40 comprise a tape tab which is preferably made of a backing laminate comprised of polymeric film and nonwoven material. Moreover, the tapes 40 comprise the first fastening component, preferably the hook members. The hook members can be formed integrally with the tape tab or can be separate pieces joined to the tape tab. Preferably, the free, distal portion of the tape 40 comprises the hook members, wherein the hook members are disposed on the inward, user facing side of the tape tab.

Alternatively, the fastening system may be an adhesive fastening system wherein the tapes comprise an adhesive layer provided at least in the free distal portion on the inward, user facing side of the tape tab. In use, the adhesive layer is attached to the landing member 42. In this embodiment, the landing member 42 comprises only a polymeric film.

The fastening system can also be a combination of a mechanical and an adhesive fastening system.

The tapes have a longitudinal width W 1 extending generally parallel to the longitudinal axis 100 and further have a transverse length L3 extending generally parallel to the transverse axis 110. The tapes have a longitudinal width W1 from 28 mm to 40 mm, preferably from about 29 mm to 35 mm, more preferably from 30 to 32 mm. The transverse length of the tape L3 is preferably from 40 mm to 70 mm, more preferably from 45 mm to 50 mm. The tapes 40 are preferably rectangular in shape. The tapes of the present invention are non-elastic, preferably highly non-elastic and still more preferably they are also non-extensible. The tapes preferably extend beyond the outermost longitudinal edge line 150 of the back ear panel 45, 46 by 25 mm to 35 mm.

The landing member has a transverse width W3 extending generally parallel to the transverse axis 110 and further have a longitudinal height H1 extending generally parallel to the longitudinal axis 100. The landing member 42 preferably has a width W3 from 140 mm to 240 mm (depending on the dimension of the chassis 22 and the size of the diaper 20). The height H1 of the landing member 42 is preferably from 34 mm to 60 mm, more preferable from 40 mm to 50 mm and still more preferable from 43 mm to 50 mm. To provide improved comfort for the caretaker when applying the diaper onto the wearer, the height H 1 of the landing member 42 should extend the width W 1 of the tapes by 8 mm to 14 mm, more preferably by 10 mm to 12 mm.

The landing member is preferably rectangular. However, if the landing member is not rectangular, the maximum transverse width and the maximum longitudinal height are taken as the transverse width W3 and the longitudinal height H1 of the landing member. The landing member of the present invention is preferably non-elastic, more preferably highly non-elastic and even more preferably is also non-extensible.

To improve the fit of the diaper around the waist and hip of the wearer, disposable diapers are often provided with elastic elements, especially with elastically stretchable back ear panels.

A way to produce non-elastic diapers is to provide a chassis and cutting out the leg openings in the crotch area of the article. Hence, the ear panels of the diaper have been an extension of the chassis and have typically been integral parts of the backsheet and/or topsheet (so-called "unibody" diapers).

Moving from non-elastic diapers to diapers with elastically stretchable ear panels, it is either possible to join an elastic member to the integral back ear panels of a unibody diaper. However, it is often desirable to modify the diaper such that the ear panels are no longer integral parts of the non-elastic backsheet and/or topsheet. In these embodiments, the ear panels are separate parts made of elastic material, which are attached to the longitudinal edges of the chassis in the back and/or front waist region. Such diapers are sometimes referred to as "multi-piece" diapers. If changing from a unibody diaper to a multi-piece diaper, the manufacturing process needs to be substantially modified.

As in unibody diapers the leg openings are cut out of the chassis, the length of the ear panels depends on how much material is cut away. Due to this manufacturing technique, small ear panels require the cut out of larger leg openings and hence, result in more waste material. Consequently, manufacturing expenses cannot be reduced by having smaller ear panels but are indeed rather increased as more waste material has to be disposed.

Contrary thereto, in multi-piece diapers principally the amounts of material required can be considerably reduced by having smaller ear panels, because the ear panels are separate material pieces attached to the chassis. However, elastically stretchable materials are typically more expensive compared to non-elastic materials. Therefore, the cost saving due to reduced amounts of material will at least partially be balanced by the requirement for more expensive material. Therefore, the overall cost saving in manufacturing may be little or the manufacturing costs may be even increased.

Hence, to further reduce the manufacturing costs, the diaper of the present invention has been provided with non-elastic back ear panels. To avoid having waste material by cutting out the leg openings from a chassis as has been the case in "unibody" diapers, the non-elastic back ear panels of the present invention are separate elements, which are joined to the chassis.

By attaching the back ear panels as separate pieces to the chassis, it is possible to cost-efficiently produce the diaper with no waste material resulting from the leg cut-out.

Moreover, by using the same general concept of a multi-piece manufacturing process for a diaper with a rectangular chassis and attached ear panels, it is possible to more efficiently exploit the manufacturing lines and to substantially increase flexibility of production: A diaper production line can now be used both for the manufacture of elastically stretchable diapers and for non-elastic diapers mainly by converting the module, which supplies the ear panel pieces. Contrary thereto, it is almost impossible to use one and the same diaper production line for the manufacture of "multipiece" diapers and "unibody" diapers.

The shape of the back ear panels can take any suitable form. To further reduce the manufacturing costs of the diaper, the size of the back ear panels should be as small as possible. However, it has been found that compared to non-elastic unibody diapers, diapers with non-elastic back ear panels of reduced length have an increased tendency to sag and droop when applied onto the wearer.

The tapes of the present invention have a width W 1 of from 28 mm to 40 mm, preferably from 29 mm to 35 mm, more preferably from 30 to 32 mm. It has been found, that the tapes do not need to have a complex ―and hence, more expensive- shape in order to provide good fit but that rectangular shaped tapes are sufficient. Moreover, the tapes can be attached to the back ear panel such that they extend at a right angle (90°) outwardly from the back ear panels. If the diaper is applied to a wearer, the tapes are fastened to the landing member in the front waist region and thus create a "tension band" extending circumferentially around the waist and hip of the wearer.

By using tapes of such dimensions, it is possible to have relatively small non-elastic back ear panels (i.e. back ear panels having a relatively small length L2) and achieving good fit comparable to the fit of larger non-elastic back ear panels having smaller tapes. Hence, a diaper with reduced sagging can be manufactured at reduced cost.

The back ear panels have a longitudinal length L2 extending generally parallel to the longitudinal axis 100 of the diaper. The length L2 of the back ear panels varies depending on the size of the diaper and the length of the chassis: Typically, the length of the back ear panels L2 varies from about 75 mm to about 145 mm. The width of the back ear panels typically is from about 60 mm to about 95 mm. The length is measured while the back ear panel is attached onto the chassis and is measured along the line where the back ear panel is joined to the chassis. The length L2 of the back ear panel is measured while the diaper is in its flat out, un-contracted state (i.e., without elastically induced contraction after the leg elastics have been cut through).

According to the present invention, relatively small non-elastic back ear panels can be used, wherein the ratio of back ear panel length L2 to chassis length L1 is from 0.18 to 0.25, more preferably from 0.18 to 0.23. This range is applicable to diapers of all sizes.

The material used for the non-elastic back ear panels should have enough strength to reduce the risk of tearing, especially during application of the diaper onto the wearer. Preferably, the back ear panels should have a Tensile Strength at Peak value of at least 4 N/cm, when measured according to the EDANA test method 20.2-89 of 2002. More preferably the Tensile Strength at Peak should be from 4 N/cm to 40 N/cm, even more preferably from 8 N/cm to 28 N/cm. Materials with such Tensile Strength at Peak values provide the necessary strength.

Furthermore, the material should comprise a relatively high opacity in order avoid shine through of the skin under the non-elastic back ear panels at the sides of the wearer. Shine through of skin through the ear panels has been found to be less appealing to consumers compared to opaque materials.

If the diaper also comprises a pair of front ear panels, the front ear panels may have the same dimensions and shape as the back ear panels. However, typically the front ear panels will be smaller in size. Further, the front ear panels can be made of the same material as the back ear panels. Preferably, the front ear panels will be made of nonwoven, more preferably of spunbond nonwoven. The basis weight of the front ear panels is preferably less than the basis weight of the back ear panels. The front ear panels are also non-elastic and preferably are highly non-elastic.

To further reduce the manufacturing expenses, the diaper of the present invention could be considered to have no elastic waist features, such as an elastic waist band. Hence, the diaper of the present invention is preferably completely non-elastic (with the exception of the leg elastics).

In substantially non-elastic diapers it is further desirable to have an optimized configuration of the diaper dimensions with respect to easy application of the diaper onto the wearer. In diapers with elastic elements, such as elastically stretchable back ear panels, application and fit of the diaper can be adjusted within certain ranges via the elastic stretchability of the back ear panels. In non-elastic diapers this is not possible.

It has been found that ease of application and comfort for the wearer can be optimized if the non-elastic diaper with back ear panels having a relatively small length dimension L2 has the following configuration:

The diaper has a maximal lateral width W2 extending from the outermost lateral edge line 150 of the first ear panel across the first back ear panel, the chassis and the second back ear panel to the outermost lateral edge line 150 of the second ear panel. The lateral width W2 of the diaper is determined along the end edges 120 of the chassis and the end edges 160 of the back ear panels. Preferably, the lateral width W2 is from 270 mm to 450 mm, depending on the size of the diaper.

The landing member comprised by the fastening system of the diaper and disposed on the front waist region, has a lateral width W3. The ratio between W3 and W2 is preferably from 0.52 to 0.70, more preferably from 0.55 to 0.65. Diapers with such configuration provide ease of application as they can be adjusted easily and well to the wearer.

### Fit studies

### Test diapers

As comparative example 1, PAMPERS diaper "Sleep & Play" Size 4 (Maxi) has been chosen, which is a unibody diaper. PAMPERS "Sleep & Play" do have non-elastic ear panels which are an integral part of the backsheet and topsheet. The back ear length of this diaper is determined by measuring the length of the ear from the end edge of the chassis in the back waist region to the point, where the leg cut out passes into a line parallel to the longitudinal axis of the diaper. The diaper is available from the Procter & Gamble Service GmbH, 65824 Schwalbach, Germany.

As a further example, comparative example 2, the multi-piece diaper PAMPERS Baby Dry (not yet on the market), Size 4 has been taken. The diaper (in its unmodified form) is available from the Procter & Gamble Service GmbH, 65824 Schwalbach, Germany. This diaper has been modified in that the elastic back ear panels have been replaced by back ear panels being non-elastic and non-extensible and consisting of 50 gsm spunbond nonwoven made of polypropylene. In the comparative example 2, the fastening tapes of the original PAMPERS Baby Dry have been used, which have a width W 1 of 26 mm.

Examples 3 and 4 are diapers according to the present invention, which are also based on PAMPERS Baby Dry, wherein the elastic back ear panels have been replaced by the non-elastic and non-extensible back ear panels as described for example 2. In example 3, the tapes have been replaced by tapes having a width W1 of 30 mm, while in example 4 tapes having a width W1 of 32 mm have been provided.

The material of the tapes is the same for all examples 1 to 4. The laminate is comprised of a laminate of a polymeric film and a nonwoven.

**Table 1: Dimensions of the diapers according to the examples**

| | Comparative example 1 (PAMPERS sleep and Play; unibody) | Comparative example 2 (modified PAMPERS Baby Dry, mulitpiece, non-stretchable back ears) | Example 3 | Example 4 |
|---|---|---|---|---|
| Chassis length L1 [mm] | 499 | 499 | 499 | 499 |
| Chassis width without back ear panels joined to chassis [mm] | - | 209 | 209 | 209 |
| Back ear length L2 [mm] | 230 | 109 | 109 | 109 |
| Ratio L2/L1 | 0.46 | 0.22 | 0.22 | 0.22 |
| Tape width W 1 [mm] | 26 | 26 | 30 | 32 |
| Total tape length [mm] | 61 | 47 | 47 | 47 |
| Tape length extending beyond the outermost lateral edge of the back ear panel [mm] | 38.5 | 31.5 | 31.5 | 31.5 |
| Diaper width W2 including chassis and back ear panels [mm] | 314 | 324 | 324 | 324 |
| Landing member width W3 [mm] | 196 | 196 | 196 | 196 |
| Landing member height H 1 [mm] | 38 | 38 | 38 | 50 |
| Ratio W3/W2 | 0.62 | 0.60 | 0.60 | 0.60 |

### Procedure:

1. 20 mothers with babies are chosen as panelists. The babies have a size suitable to wear a diaper size 4. Moreover, the babies must be able to stand on their own. The baby panelists are selected to consist of 10 girls and 10 boys.

**Table 2: The baby panelists shall have the following weight split:**

| | | | | | |
|---|---|---|---|---|---|
| # of babies | 3 | 5 | 5 | 4 | 3 |
| Weight | 7 - 9 kg | 9 - 11 kg | 11 -13 kg | 13 - 14 kg | > 14 kg |

2. The mothers apply the test diapers they to their babies. After the diaper is applied, the following measurements are taken on the standing baby:
a) The shortest distance between the baby's navel and the upper edge of the landing zone in the front waist region is measured.
b) The point on the back of the baby, which's height corresponds to the position of the baby's navel in the front is determined. Then a point about 5 cm above this point is taken, gently market on the baby's skin and the shortest distance between this market point on the babies back and the end edge of the chassis in the back waist region is determined. The end edge of the chassis should not be folded downwards when the measurement is taken.
Principally, for carrying out this test, the exact location of the reference points do not matter as long as it is ensured that the same reference points are taken for the initial measurement and for the measurement at the end of the test.
3. The diaper is loaded with 150 ml saline solution. To best simulate "natural" conditions, the diapers are loaded as follows: For girls, the diaper is loaded at a point which is 13 cm below the end edge of the absorbent core in the front waist region. For boys, the diaper is loaded at a point which is 10 cm below the end edge of the absorbent core in the front waist region.
4. After the diapers are loaded, the babies move and play for 30 minutes.
After 30 minutes, the same measurements as set out under point 2 (a and b) are taken again. The degree of slippage/sagging in the front and back region is determined by subtracting the initial value before loading the diaper from the value taken after 30 minutes.
Each panelist tests all 4 diaper examples.

**Table 3: Results**

| Fit Study Results | Comparative example 1 (unibody) | Comparative example 2 (multi-piece, non-elastic back ears, tape width W1 26 mm) | Example 3 (multi-piece, non-elastic back ears, tape width W1 30 mm) | Example 4 (multi-piece, non-elastic back ears, tape width W1 32 mm) |
|---|---|---|---|---|
| Front Slippage [mm] | 21 | 31 | 25 | 24 |
| Back Chassis Slippage [mm] | 16 | 24 | 16 | 14 |

As can be seen from the results, the degree of slippage of examples 3 and 4 is comparative to the degree of slippage of the unibody diaper of example 1, which has relatively large back ear panels. Contrary thereto, the diaper of example 2, which is the multi-piece diaper with conventional dimensions as used in multi-piece diapers with stretchable ear panels, shows considerably increased sagging.

## Claims

1. A disposable diaper (20) having a front waist region (36), a back waist region (38) and a crotch region (37) between the front waist region (36) and the back waist region (38), wherein the diaper comprises:
a chassis (22) having longitudinal edges (130) and end edges (120), the chassis comprising a liquid pervious topsheet (24), a liquid impervious backsheet (26) associated with the topsheet (24), and an absorbent core (28) disposed between the topsheet (24) and the backsheet (26); and
the diaper further comprising first and second back ear panels (45, 46) extending laterally outward from the chassis (22) in the back waist region (38), each of the back ear panels (45, 46) having an innermost longitudinal edge line (140) and an outermost longitudinal edge line (150); each of the back ear panels (45, 46) being joined along its innermost longitudinal edge line (140) to a respective longitudinal edge (130) of the chassis (22), each of the back ear panels (45, 46) being non-elastic,
wherein the chassis (22) has a longitudinal length L1 and the first and second back ear panel (45, 46) each have a longitudinal length L2,
the diaper further comprising a fastening system comprising a pair of tapes (40), each tape having a longitudinal width W1, wherein one tape is attached to the first back ear panel (45) and the other tape is attached to the second back ear panel (46), the tapes (40) extending laterally outward from the back ear panels (45, 46) beyond the outermost longitudinal edge line (150) of the back ear panels (40),
**characterized in that** each tape (40) has a width W1 of from 28 mm to 40 mm and further **characterized in that** the ratio of L2 to L1 is from 0.18 to 0.24.

2. The diaper of claim 1 wherein the back ear panels (45, 46) have a tensile strength at peak of at least 4 N/cm.

3. The diaper of claim 1 or 2 wherein the back ear panels (45, 46) are non-extensible.

4. The diaper of any of the preceding claims wherein the tapes (40) are non-elastic.

5. The diaper of claim 4 wherein the tapes (40) are non-extensible.

6. The diaper of any of the preceding claims wherein the topsheet (24), the backsheet (26) and the absorbent core (28) are non-elastic.

7. The diaper of any of the preceding claims wherein the diaper has a lateral width W2 and wherein the fastening system further comprises a landing member (42) attached to the backsheet (26) in the front waist region (36) and the landing member (42) having a transverse width W3, wherein the ratio of W3 to W2 is from 0.52 to 0.70, preferably from 0.55 to 0.65.

## Patentansprüche

1. Einwegwindel (20) mit einem vorderen Taillenbereich (36), einem hinteren Taillenbereich (38) und einem Schrittbereich (37) zwischen dem vorderen Taillenbereich (36) und dem hinteren Taillenbereich (38), wobei die Windel Folgendes umfasst:
eine Grundeinheit (22) mit Längsrändern (130) und Endrändern (120), wobei die Grundeinheit eine flüssigkeitsdurchlässige Oberschicht (24), eine flüssigkeitsundurchlässige Unterschicht (26), die mit der Oberschicht (24) verbunden ist, und einen Absorptionskern (28), der zwischen der Oberschicht (24) und der Unterschicht (26) angeordnet ist, umfasst; und
wobei die Windel ferner ein erstes und ein zweites hinteres Flügelfeld (45, 46) umfasst, die sich im hinteren Taillenbereich (38) von der Grundeinheit (22) in Seitenrichtung nach außen erstrecken, wobei jedes der hinteren Flügelfelder (45, 46) eine innerste Längsrandlinie (140) und eine äußerste Längsrandlinie (150) aufweist; wobei jedes der hinteren Lappenfelder (45, 46) entlang seiner innersten Längsrandlinie (140) mit einem jeweiligen Längsrand (130) der Grundeinheit (22) verbunden ist, wobei jedes der hinteren Flügelfelder (45, 46) nichtelastisch ist,
wobei die Grundeinheit (22) eine Längsausdehnung L1 aufweist und das erste und das zweite hintere Flügelfeld (45, 46) jeweils eine Längsausdehnung L2 aufweisen,
wobei die Windel ferner ein Befestigungssystem umfasst, das ein Paar Streifen (40) umfasst, wobei jeder Streifen eine längs verlaufende Breite W1 aufweist, wobei ein Streifen am ersten hinteren Flügelfeld (45) befestigt wird und der andere Streifen am zweiten hinteren Flügelfeld (46) befestigt wird, wobei sich die Streifen (40) von den hinteren Flügelfeldern (45, 46) über die äußerste Längsrandlinie (150) der hinteren Flügelfelder (40) in Seitenrichtung nach außen erstrecken,
**dadurch gekennzeichnet, dass** jeder Streifen (40) eine Breite W1 von 28 mm bis 40 mm aufweist, und ferner **dadurch gekennzeichnet, dass** das Verhältnis von L2 zu L1 von 0,18 bis 0,24 beträgt.

2. Windel nach Anspruch 1, wobei die hinteren Flügelfelder (45, 46) eine Spitzenzugfestigkeit von mindestens 4 N/cm aufweisen.

3. Windel nach Anspruch 1 oder 2, wobei die hinteren Flügelfelder (45, 46) nicht dehnbar sind.

4. Windel nach einem der vorstehenden Ansprüche, wobei die Streifen (40) nichtelastisch ist.

5. Windel nach Anspruch 4, wobei die Streifen (40) nicht dehnbar sind.

6. Windel nach einem der vorstehenden Ansprüche, wobei die Oberschicht (24), die Unterschicht (26) und der Absorptionskern (28) nichtelastisch sind.

7. Windel nach einem der vorstehenden Ansprüche, wobei die Windel eine seitliche Breite W2 aufweist und wobei das Befestigungssystem ferner ein Anlegeelement (42) umfasst, das an der Unterschicht (26) im vorderen Taillenbereich (36) angeordnet ist, und das Anlegeelement (42) eine quer verlaufende Breite W3 aufweist, wobei das Verhältnis von W3 zu W2 von 0,52 bis 0,70, vorzugsweise von 0,55 bis 0,65 beträgt.

## Revendications

1. Couche jetable (20) ayant une région de ceinture avant (36), une région de ceinture arrière (38) et une région d'entrejambe (37) entre la région de ceinture avant (36) et la région de ceinture arrière (38), où la couche comprend :
un châssis (22) ayant des bords longitudinaux (130) et des bords extrêmes (120), le châssis comprenant une feuille de dessus perméable aux liquides (24), une feuille de fond imperméable aux liquides (26) associée à la feuille de dessus (24), et une âme absorbante (28) disposée entre la feuille de dessus (24) et la feuille de fond (26) ; et
la couche comprenant en outre des premier et deuxième rabats arrière (45, 46) s'étendant latéralement vers l'extérieur à partir du châssis (22) dans la région de ceinture arrière (38), chacun des rabats arrière (45, 46) ayant une ligne de bord longitudinal interne (140) et une ligne de bord longitudinal externe (150) ; chacun des rabats arrière (45, 46) étant joint le long de sa ligne de bord longitudinal interne (140) à un bord longitudinal (130) respectif du châssis (22), chacun des rabats arrière (45, 46) étant non élastique,
dans laquelle le châssis (22) a une longueur longitudinale L1 et les premier et deuxième rabats arrière (45, 46) ont chacun une longueur longitudinale L2,
la couche comprenant en outre un système de fixation comprenant une paire de rubans (40), chaque ruban ayant une largeur longitudinale W1, dans laquelle un ruban est fixé au premier rabat arrière (45) et l'autre ruban est fixé au deuxième rabat arrière (46), les rubans (40) s'étendant latéralement vers l'extérieur à partir des rabats arrière (45, 46) au-delà de la ligne de bord longitudinal externe (150) des rabats arrière (40),
**caractérisée en ce que** chaque ruban (40) a un largeur W1 allant de 28 mm à 40 mm et **caractérisée en outre en ce que** le rapport de L2 sur L1 va de 0,18 à 0,24.

2. Couche selon la revendication 1, dans laquelle les rabats arrière (45, 46) ont une résistance à la traction au pic d'au moins 4 N/cm.

3. Couche selon la revendication 1 ou 2, dans laquelle les rabats arrière (45, 46) sont non extensibles.

4. Couche selon l'une quelconque des revendications précédentes, dans laquelle les rubans (40) sont non élastiques.

5. Couche selon la revendication 4, dans laquelle les rubans (40) sont non extensibles.

6. Couche selon l'une quelconque des revendications précédentes, dans laquelle la feuille de dessus (24), la feuille de fond (26) et l'âme absorbante (28) sont non élastiques.

7. Couche selon l'une quelconque des revendications précédentes, où la couche a une largeur latérale W2 et dans laquelle le système de fixation comprend en outre un élément de réception (42) fixé à la feuille de fond (26) dans la région de ceinture avant (36) et l'élément de réception (42) ayant une largeur transversale W3, dans laquelle le rapport de W3 sur W2 va de 0,52 à 0,70, de préférence de 0,55 à 0,65.
